# EUROPEAN PATENT SPECIFICATION

(11) **EP 1 897 507 B1**
(45) Date of publication and mention of the grant of the patent: **28.07.2010**
(21) Application number: 07251843.4
(22) Date of filing: 02.05.2007
(51) Int. Cl.: A61B 17/34, A61M 1/00, A61B 19/00, A61B 10/02

(54) **Localizing obturator**
Lokalisationsobturator
Obturateur de localisation

(30) Priority: 06.09.2006 US 516277
(43) Date of publication of application: 12.03.2008
(73) Proprietor: Suros Surgical Systems, Inc., Indianapolis, IN 46278 (US)
(72) Inventor: Nicoson, Zachary R, Indianapolis, Indiana 46220 (US); Mark, Joseph L, Indianapolis, Indiana 46208 (US)
(74) Representative: Cheyne, John Robert Alexander M.

(56) References cited:
- WO-A-2006/037950
- DE-A1- 10 337 368
- US-A- 4 177 814
- US-A1- 2003 233 101
- US-A1- 2004 077 938
- US-B1- 6 272 372
- US-B1- 6 628 982

## Description

### BACKGROUND OF THE INVENTION

### Field of the Invention

The present invention relates to the field of medical devices and more particularly to a medical system for introducing, among other things, minimally invasive surgical instruments and other medical treatments into a patient's body.

### Description of the Related Art

Medical procedures have advanced to stages where less invasive or minimally invasive surgeries, diagnostic procedures and exploratory procedures have become desired and demanded by patients, physicians, and various medical industry administrators. To meet these demands, improved medical devices and instrumentation have been developed, such as cannulas or micro-cannulas, medical introducers, vacuum assisted biopsy apparatus, and other endoscopic related devices.

In the field of tissue biopsy, minimally invasive biopsy devices have been developed that require only a single insertion point into a patient's body to remove one or more tissue samples. One such biopsy device incorporates a "tube-within-a-tube" design that includes an outer piercing needle having a sharpened distal end and a lateral opening that defines a tissue receiving port. An inner cutting member is slidingly received within the outer piercing needle, which serves to excise tissue that has prolapsed into the tissue receiving port. A vacuum is used to draw the excised tissue into the tissue receiving port and aspirates the excised tissue from the biopsy site once severed.

Exemplary "tube-within-a-tube" biopsy devices are disclosed in pending U.S. Patent Applications, Serial Nos. 09/707,022 and 09/864,031, which are owned by the assignee of the present invention. Among other features, the exemplary biopsy devices can be used in conjunction with Magnetic Resonance Imaging (MRI). This compatibility is due to the fact that many of the components of the biopsy devices are made of materials that do not interfere with operation of MRI apparatus or are otherwise compatible therewith. It is desirable to perform biopsies in conjunction with MRI because it is currently the only non-invasive visualization modality capable of defining the margins of a tumor.

While the exemplary MRI compatible biopsy devices have proven effective in operation, in some procedures it is desirable to create a pathway to the biopsy site for precise introduction of the biopsy device and other medical treatments into the patient. For these and other reasons, an MRI compatible medical introduction system is desirable for use with minimally invasive biopsy devices, such as those employing a "tube-within-a-tube" design.

### SUMMARY OF THE INVENTION

A medical target confirmation device, such as a localizing obturator, is disclosed. In one embodiment, the medical target confirmation device includes an elongate body member defined by a distal end and a proximal end. The distal end includes at least one bore extending therein. The bore receives contrast agent therein. A method for using the medical target confirmation device is also disclosed.

### BRIEF DESCRIPTION OF THE DRAWINGS

Embodiments of the invention will now be described, by way of example, with reference to the accompanying drawings, wherein:
FIG. 1 is a side view of an introducer stylet according to an embodiment of the present invention;
FIG. 2 is side view of an outer cannula and fluid conduit according to an embodiment of the present invention;
FIG. 3 is a side view of a target confirmation device according to an embodiment of the present invention;
FIGS. 3A and 3B are side views of a target confirmation device according to alternate embodiments of the present invention;
FIG. 3C is a perspective view of a localizing obturator according to an embodiment of the present invention;
FIG. 3D is a close-up view of area 3D, E, F of the localizing obturator of FIG. 3C.
FIG. 3E is a close-up view of area 3D, E, F of an alternative embodiment of the localizing obturator of FIG. 3C.
FIG. 3F is a close-up view of are 3D, E, F of yet another alternative embodiment of the localizing obturator of FIG. 3C.
FIG. 4 is a side view of an exemplary biopsy device for use with the introduction system of the present invention;
FIG. 5 is a detailed cross-sectional view of a cutting element of the biopsy device of FIG. 4;
FIG. 6 is a side view of an aspiration wand suitable for insertion into the outer cannula; and
FIGS. 7-11 are elevational views illustrating a medical procedure using the medical system of the present invention.

### DETAILED DESCRIPTION

Referring now to the drawings, the preferred illustrative embodiments of the present invention are shown in detail. Although the drawings represent some preferred embodiments of the present invention, the drawings are not necessarily to scale and certain features may be exaggerated to better illustrate and explain the present invention. Further, the embodiments set forth herein are not intended to be exhaustive or otherwise limit or restrict the invention to the precise forms and configurations shown in the drawings and disclosed in the following detailed description.

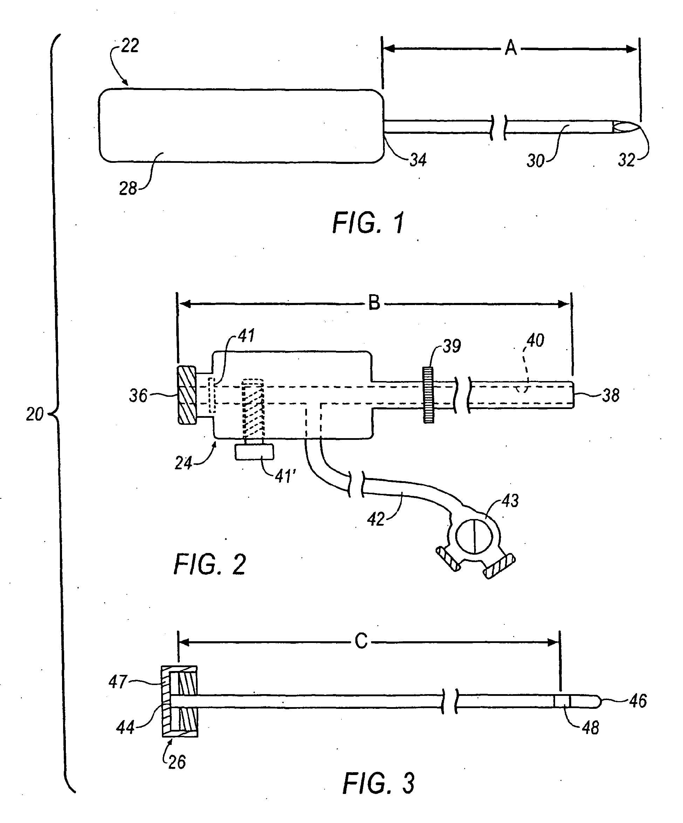

Referring to FIGS. 1-3, a medical system 20 is shown that includes an introducer stylet 22, an outer cannula 24 and a target confirmation device 26. As will be described in detail, system 20 is particularly, but not necessarily, suited for use in biopsy procedures that identify the target biopsy site using Magnetic Resonance Imaging (MRI) or comparable medical imaging modality.

In an embodiment, introducer stylet 22 includes a handle 28 and a stylet 30 having a distal end 32 and a proximal end 34 connected to handle 28. Handle 28 may be made of a medical grade resin or other MRI compatible material. Stylet 30 may also be made of an MRI compatible, medical grade material, such as 316 stainless steel or inconel 625.

In a particular configuration, a distal end 32 of stylet 30 includes a tissue piercing tip, such as a trocar tip, to facilitate penetration of stylet 30 into a patient's tissue. In addition to a trocar tip, it will be appreciated that stylet 30 may include other devices for of piercing the patient's tissue, including without limitation, devices that use a laser or radio frequencies (RF) to pierce the tissue. The length of stylet 30 is generally denoted by the reference character "A" in FIG. 1.

Referring to the embodiment shown in FIG. 2, outer cannula 24 extends from an open proximal end 36 to an open distal end 38, which is separated from proximal end 36 by a distance "B." Like introducer stylet 30, outer cannula 24 may be made from a medical grade resin or other MRI compatible material. In some configurations, proximal end 36 may include a luer-style fitting or other suitable configuration for interfacing, but not necessarily connecting, outer cannula 24 with target confirmation device 26. A depth limiting member 39, such as a rubber o-ring, may be moveably disposed on outer cannula 24 to limit the insertion depth of outer cannula 24 into the patient's body.

In an embodiment, outer cannula 24 also includes an inner lumen 40 therethrough, which is open to communication with a fluid conduit 42 for supplying fluids, such as saline and anesthetics, or removing fluids, such as blood, from the patient's body. Fluid conduit 42 communicates with inner lumen 40 via a port in outer cannula 24. In some configurations, outer cannula 24 may include a haemostatic valve, depicted generally as element 41, or a manually operable valve 41' that can be selectively closed to prevent the escape of fluid from proximal end 36. Fluid conduit 42 may also include a directional valve 43 to selectively control the supply and removal of fluid to and from inner lumen 40, respectively.

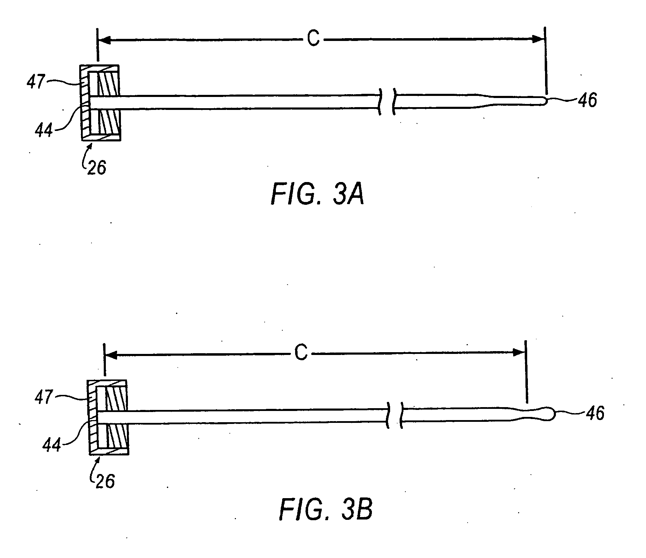

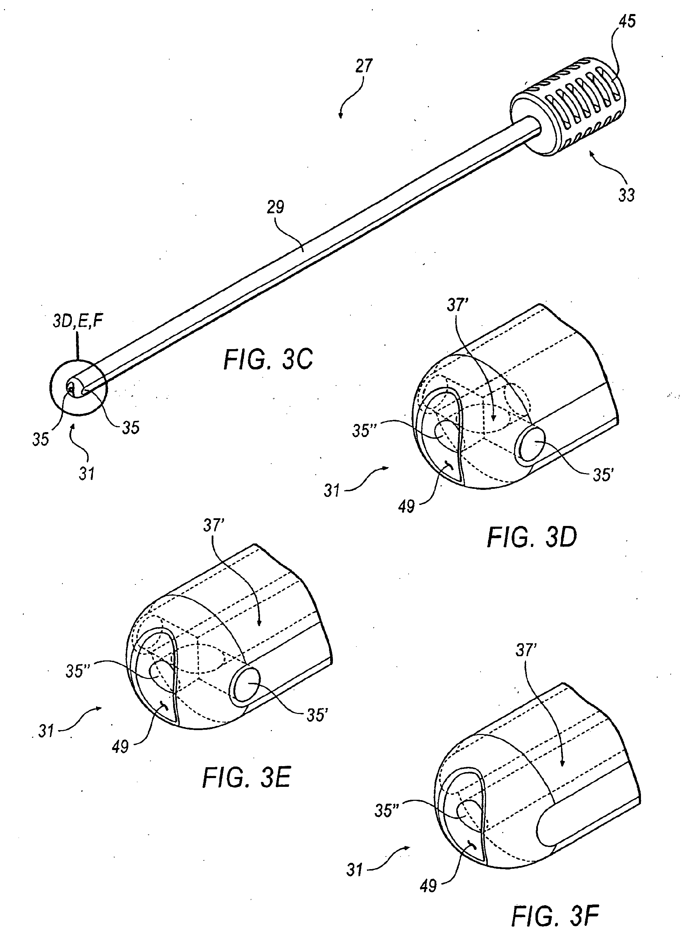

In the embodiment shown in FIG. 3, target confirmation device 26 is an elongated member that is sized to fit within inner lumen 40 of outer cannula 24. Target confirmation device 26, which may be made of a medical grade resin or other MRI compatible material, extends from a connecting end 44 to a distal end 46. Connecting end 44 may be configured with a cap 47 that abuts outer cannula 24. In some configurations, cap 47 may include a luer-style fitting or other suitable feature for interfacing, but not necessarily connecting, target confirmation device 26 with outer cannula 24.

Distal end 46 of target confirmation device 26 is generally rounded to facilitate entry into the patient's body. In an embodiment, a portion of target confirmation device 26 is configured with a magnetic resonance imaging (MRI) identifiable material, such as inconel 625, titanium or other material with similar magnetic characteristics. In one particular configuration, a targeting band 48 is provided a distance "C" from connecting end 44, as shown in FIG. 3; the distance C being measured from the approximate center of targeting band 48 to connecting end 44 (or the inside of cap 47), for example. Targeting band 48 provides a reference point in an MR image relative to the target biopsy tissue.

In another embodiment of the present invention, the tip of target confirmation device itself may be used to provide the reference point in the MR image, provided the target confirmation device material exhibits a relatively low artifact during MR imaging. As used herein, the term "artifact" describes a material's tendency to distort an MR image. A material exhibiting a relatively high artifact will render the body tissue surrounding the material unreadable in an MR image. Conversely, a material with a relatively low artifact will allow the material to be readily identified in the MR image and will not significantly distort the MR image of the surrounding tissue.

As shown in the embodiments of FIGS. 3A and 3B, the distal end 46 of target confirmation device 26 may include a particular shape to help identify the location of target confirmation device 26 relative to the surrounding tissue. In the embodiment of FIG. 3A, a portion of target confirmation device 26 adjacent the distal end 46 has a smaller diameter relative to the remaining length. In the embodiment of FIG. 3B, a portion of target confirmation device 26 is tapered to provide an hour glass like image when viewed under MR. It will be appreciated that the embodiments represented in FIGS. 3A and 3B are not limited to the configurations shown, and that other configurations are with in the scope of the present invention.

FIGS. 3C-3D illustrate an embodiment of a localizing obturator 27 that may be used as a target confirmation device. Obturator 27 includes an elongate body 29 defined by a distal end 31 and proximal end 33. Elongate body 29 is sized to so as to fit within inner lumen 40 of outer cannula 24.

Distal end 31 of obturator 27 may be generally rounded to facilitate entry into the patient's body. In the embodiment shown in FIGS. 3C and 3D, distal end 31 is provided with one or more bores 35 formed in distal end 31. In one embodiment, there are at least two such bores, a generally lateral bore 35' and a generally axial bore 35", which intersect one another to form an internal reservoir 37, to be explained further below. While the intersection of bores 35 may be at any angle, in one embodiment, cores 35 intersect one another at approximately 90°. While generally lateral bore 35' is shown as extending completely through distal end 31 of obturator 27, it is also understood that one or end of bore 35' may be closed. In yet another alternative embodiment, generally axial bore 35" may open into a blind bore 35' where by both ends of bore 35' are closed.

In the embodiment shown in FIG. 3D, axial bore 35" has a predetermined length that extends a predetermined distance beyond generally lateral bore 35'. In another alternative embodiment, as shown in FIG. 3E, axial bore 35" extends through the length of elongate body 29, so as to form an elongated reservoir 37 that terminates at proximal end 33. In yet another alternative embodiment, as shown in FIG. 3F, only a single bore 35" is formed at distal end 31. Single bore 35" extends the length of elongate body 29 to form an elongated reservoir 37 that terminates at proximal end 33. An intersecting bore (not shown) may also be provided adjacent to proximal end 33.

Proximal end 33 is sized so as to be larger than inner lumen 40 of cannula 24 such that the entire obturator 27 may not be delivered into a patient's body. In one embodiment, proximal end 33 includes a number of gripping depressions 45 to assist a user in gripping obturator 27. Alternatively, proximal end 33 may include a cap, such as described above in connection with FIG. 3.

To assist in imaging a target site, a contrast agent is introduced into the bores 35' 35" of obturator 27. In the embodiment shown in FIG. 3D, this may be accomplished by dipping distal end 31 into a contrast agent. Bores 35 permit the contrast agent to "wick" into the bores 35 and be held in the reservoir. Alternatively, contrast agent may be injected into bores 35', 35". Further, bores 35' and 35" may also be provided with plugs to seal contrast agent in reservoir 37.

After the contrast agent has been introduced into reservoir 37, when obturator 27 is placed into the body via outer cannula 24, the contrast agent is visible. Suitable contrast agents include fluro-deoxyglucose (FDG), technicium 99 or other similar radioactive isotope. These radioactive isotopes are visible under imaging modalities such as PET (positron emission tomography), gamma cameras, or scintimammography. The radioactive isotopes attach to glucose, such that highly active cells (typically cancer) metabolize the glucose much more rapidly than normal tissue cells. Thus, the contrast agent is concentrated in the areas of high metabolic activities and shows up as bright areas under the imaging modalities.

In operation, after the contrast agent is introduced into the reservoir, either by dipping or by injection, at least a portion of the contrast agent is retained within the reservoir 37. Next, obturator 27 is inserted into inner lumen 40 of outer cannula 24. As obtuator 27 is inserted therein, distal end 31 passes through hemostatic valve 41. Because a portion of the contrast agent is retained within the reservoir 37, the contrast material will still be visible under the imaging modalities even if the frictional force between the hemostatic valve 41 and the distal end 31 of the obturator 27 wipes some of the contrast material off the obturator 27 outside surface. Further, in one embodiment, distal end 31 may be formed with an inwardly extending depression 49 that substantially surrounds bore 35. Depression 49 further serves to reduce the likelihood that the contrast material will be removed from obturator 27. The visibility of the contrast agent is also significant as the contrast material that has wicked into the bores 35 view is pure contrast agent in that it has not been metabolized by in the surrounding tissue and thus has not be diluted. Once the obturator 27 has been placed in the body, the contrast agent will be easily visible under the imaging modalities, thereby indicating a target site where a biopsy instrument may be placed.

In still another embodiment, introducer stylet 30 may function as a target confirmation device. In this embodiment, introducer stylet 30, and more particularly stylet 30, may be made of an MRI compatible material that preferably, but not necessarily, exhibits a relatively low artifact.

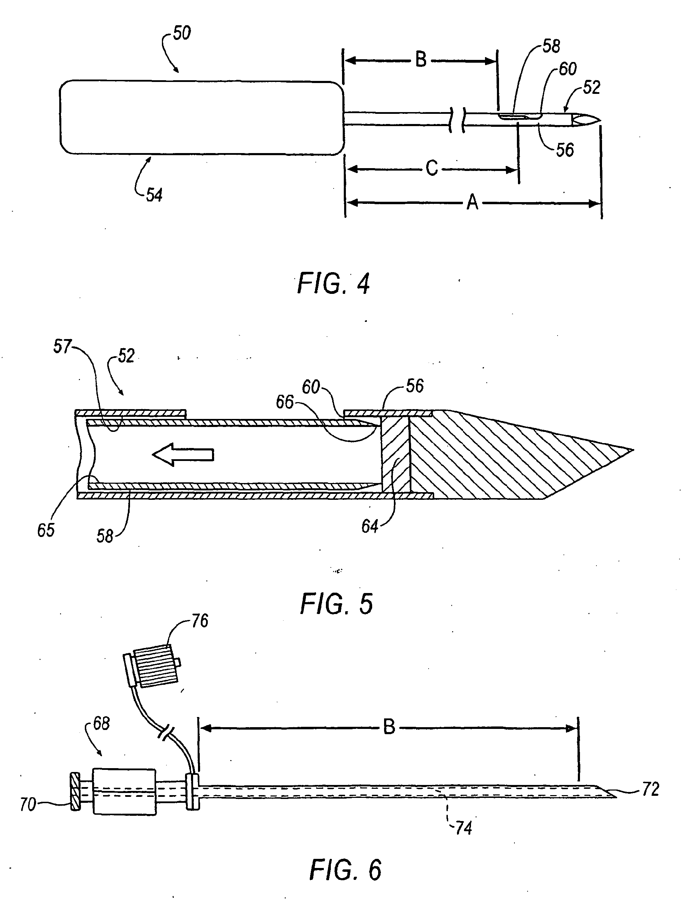

An exemplary biopsy apparatus 50, which is suitable for use with medical system 20 of the present invention, is generally shown in FIG. 4 and in more detail in FIG. 5.

Apparatus 50 includes a cutting element 52 sized for introduction into the patient's body and a hand piece 54. The exemplary biopsy apparatus 50 is configured as a "tube-within-a-tube" cutting device. More particularly, cutting element 52 includes an outer cannula 56 having an outer lumen 57 and an inner cannula 58 sized to fit concentrically within the outer lumen. A motor or other motion generating device is provided within hand piece 54 to rotate and/or translate inner cannula 58 within outer cannula 56. Biopsy apparatus similar to apparatus 50 can be seen by way of example in pending U.S. Patent Applications, Serial Nos. 09/707,022 and 09/864,03, which are owned by the assignee of the present invention and are incorporated herein by reference in their entirely.

A particular embodiment of the working end of cutting element 52 is depicted in FIG. 5. In the illustrated embodiment, outer cannula 56 defines a tissue-receiving opening 60, which communicates with outer lumen 57. The working end of cutting element 52 further includes a cutting board 64 that is disposed within outer lumen 57 at the distal end of outer cannula 56. Inner cannula 58 defines an inner lumen 65 that is hollow along its entire length to provide for aspiration of the biopsy sample (tissue). Inner cannula 58 terminates in a cutting edge 66 that may be formed by an inwardly beveled surface having a razor-sharp edge.

Referring to FIG. 6, an aspirating wand 68 is shown that can be inserted into outer cannula 24. In an embodiment, aspirating wand 68 extends from a connecting end 70 to an insertion end 72 and includes an inner lumen 74 that extends from connecting end 70 to insertion end 72. Connecting end 70 may include a luer interface or other suitable fitting for connecting aspirating wand 68 to a vacuum source (not shown). Aspirating wand 68 may also include a cap 76 that can be placed onto connecting end 70 to inhibit fluid leakage when aspirating wand 68 is inserted into the patient. The haemostatic valve 41 in outer cannula 24 seals against aspirating wand 68, as it does against target confirmation device 26 and biopsy device 50, when inserted into outer cannula 24. Additionally, the outside diameter of aspirating wand 68 is less than the inside diameter of inner lumen 40 to allow saline or other fluids introduced through fluid conduit 40 to pass into the patient's body. When cap 76 is removed and aspirating wand 68 is connected to a vacuum source, fluids, such as blood and saline, can be aspirated from the biopsy site.

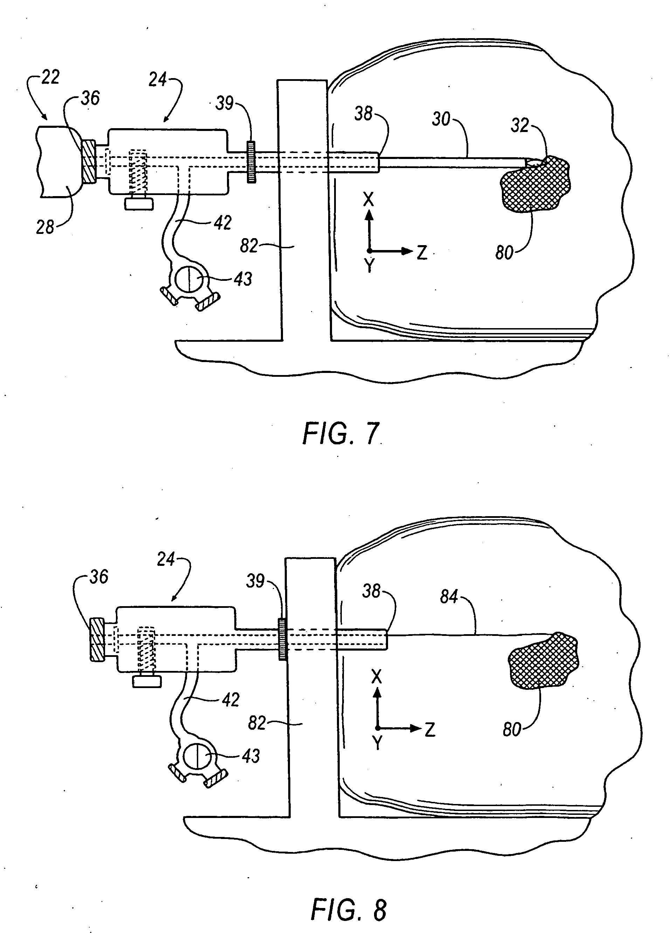

Referring to FIGS. 7-11, a medical procedure using system 20 of the present invention will be described. In an embodiment, system 20 is employed to conduct a biopsy of a lesion within a patient's body. The target tissue or lesion to be biopsied and/or removed from the patient's body (denoted generally by mass 80 in FIG. 7) is located using a medical imaging system, such as MRI or other suitable imaging modality. A reference structure 82 may be positioned adjacent the patient to assist in locating the target tissue. The location of the target tissue 80 relative to reference structure 82 may be determined along one or more axis. In the illustrated embodiment, the target tissue location relative to reference structure 82 is determined along the X and Y axes; however, the target tissue location may also be determined along all three of the X, Y and Z axes. While the described method employs a reference structure 82 to locate the target tissue, the reference structure is not necessarily required and a more "free-hand" approach may be utilized.

In an embodiment, reference structure 82 includes a support grid having a number of holes therethrough. Each hole is sized to allow passage of outer cannula 24. The hole through which outer cannula 24 is ultimately inserted is determined by the location of target tissue 80 relative to reference structure 82 along the X and Y axes. The patient and reference structure 82 are viewed using a medical imaging system, such as MRI, to determine the location of the target tissue relative to reference structure 82.

After application of anesthesia, the stylet portion of introducer stylet 22 and a portion of outer cannula 24 are inserted through the support grid and into the patient's body, creating a pathway 84 to the target tissue 80 (see, e.g., FIG. 7). Introducer stylet 22 is then removed from the patient's body leaving behind outer cannula 24 (see, e.g., FIG. 8).

Fluids may be inserted into or removed from the patient's body through inner lumen 40 via fluid conduit 42. These fluids may include, for example, additional anesthetics and/or saline solution to cleanse pathway 84 and remove blood. Accumulated blood and other fluids within pathway 84 may be aspirated through fluid conduit 42 or by inserting aspirating wand 68 prior to insertion of target confirmation device 26.

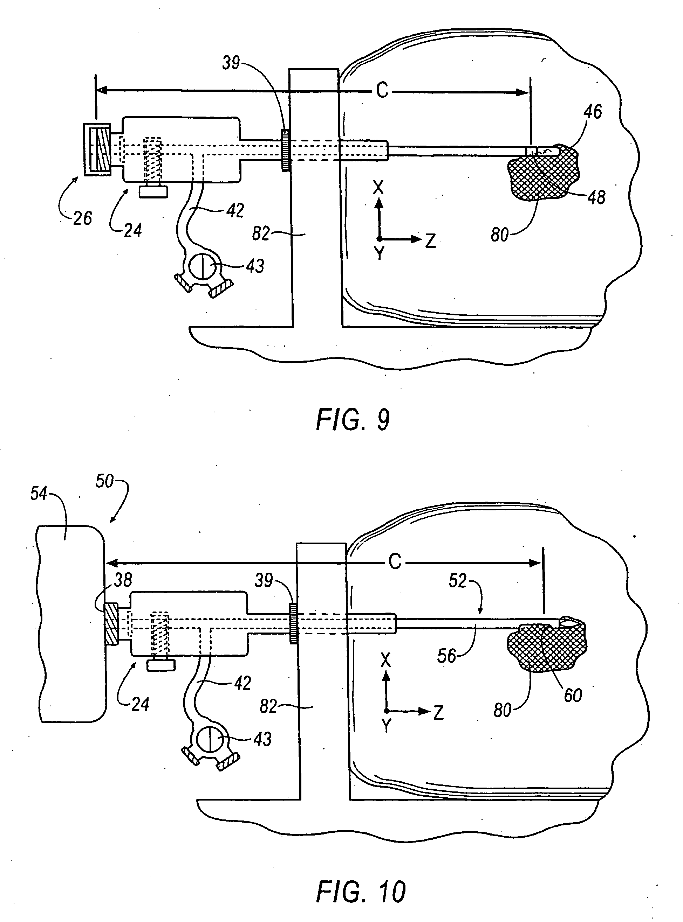

Once introducer stylet 22 is removed from outer cannula 24, target confirmation device 26 may be inserted into the patient's body through the port created by outer cannula 24 (see, e.g., FIGS. 8 and 9). With target confirmation device 26 properly inserted into outer cannula 24, an image of the target site is again taken to determine the location of targeting band 48 in relation to the target tissue and reference structure 82. If targeting band 48 is in the desired position adjacent target tissue 80 along the Z-axis, targeting device 26 is removed from outer cannula 24. However, if targeting band 48 is not in the desired position, then the position of target confirmation device 26 and outer cannula 24 is modified along the Z-axis until the desired position is achieved.

Once the desired position is achieved, depth limiting member 39 is moved against reference structure 82 to inhibit movement of outer cannula 24 further into the patient. When no reference structure 82 is used, depth limiting member may be moved directly against the patient's skin. Target confirmation device 26 is then removed from outer cannula 24 and biopsy device 50 is inserted into outer cannula 24 until handpiece 54 abuts proximal end 36 of outer cannula 24. In the embodiment illustrated in FIG. 10, one or more samples of target tissue 80 are removed from the patient through tissue-receiving opening 60. The correct position of tissue-receiving opening 60 is ensured because the distance "C" between proximal end 44 of target confirmation device 26 and targeting band 48 (see, e.g., FIGS. 3 and 9), or the distance between proximal end 44 and the predetermined location on target confirmation device 26 (FIGS. 3A and 3B), is approximately equal to the distance between the center of tissue receiving opening 60 and handpiece 54 of biopsy device 50.

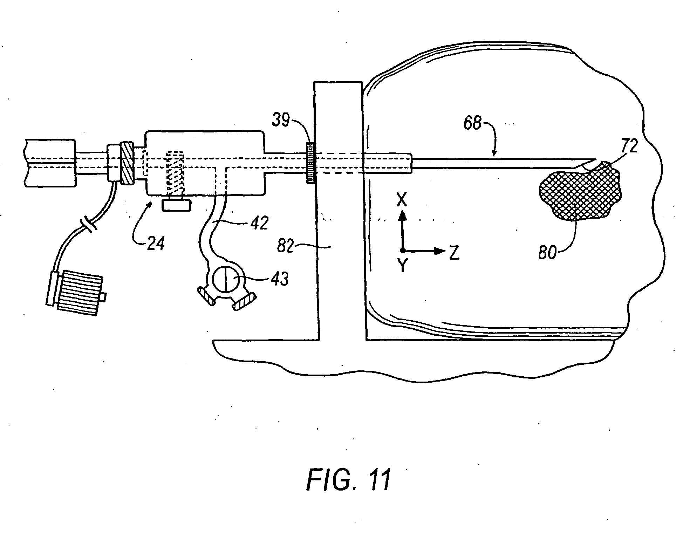

After completion of the biopsy, the biopsy site can be aspirated using aspirating wand 68 (see, e.g., FIG. 11). During or after aspiration, a final image of the biopsy site can be taken to confirm removal of the target tissue. Finally, an identifiable marker, such as a collagen plug, or other medical treatment can be inserted into the biopsy site through outer cannula 24.

Among other features, the medical system of the present invention localizes the target biopsy site in a manner that allows confirmation of the target biopsy site under MRI or other visualization modality, and allows positioning of a biopsy device to ensure the cutting element of the biopsy device can be accurately placed at the target biopsy site. The medical system of the present invention also facilitates the introduction and removal of fluids from the target site, including without limitation, anesthesia and blood, but minimizes the exposure of the fluids to the adjacent equipment and medical staff. In addition to allowing the medical staff to identify the presence of significant bleeding and to introduce a biopsy device into the patient, the medical system provides access to the target site to introduce a medical treatment, such as a site marker, tamponade or other haemostatic agent, after removal of the tissue.

The present invention has been particularly shown and described with reference to the foregoing embodiments, which are merely illustrative of the best modes for carrying out the invention. It should be understood by those skilled in the art that various alternatives to the embodiments of the invention described herein may be employed in practicing the invention without departing from the scope of the invention as defined in the following claims. It is intended that the following claims define the scope of the invention and that the method and apparatus within the scope of these claims and their equivalents be covered thereby. This description of the invention should be understood to include all novel and non-obvious combinations of elements described herein, and claims may be presented in this or a later application to any novel and non-obvious combination of these elements. Moreover, the foregoing embodiments are illustrative, and no single feature or element is essential to all possible combinations that may be claimed in this or a later application.

## Claims

1. A medical targeting device (27) for a target confirmation procedure, comprising:
an elongate body member (29) defined by a distal end (31) and a proximal end (33);
wherein said distal end (31) includes at least one bore (35, 35', 35") extending therein;
**characterised in that** the bore contains a fluid contrast agent which is received in the bore (35, 35', 35") by one of wicking or injecting.

2. The medical targeting device (27) of claim 1, wherein said distal end (31) includes two bores (35', 35") formed therein.

3. The medical targeting device (27) of claim 2, wherein the bores (35', 35") intersect one another.

4. The medical targeting device (27) of claim 3, wherein the bores (35', 35") intersect one another at an approximately 90° angle.

5. The medical targeting device (27) of claim 4, wherein the bores (35', 35") form a cross-shaped passage.

6. The medical targeting device (27) of claim 4, wherein said bores (35', 35") form a t-shaped passage.

7. The medical targeting device (27) of claim 1, wherein one bore (35") extends substantially the length of said body (29).

8. The medical targeting device (27) of claim 7, further including a plug to selectively seal said bore (35, 35', 35 "), closed so as to seal said contrast agent in said medical targeting device.

9. The medical targeting device (27) of claim 1, wherein said proximal end (33) includes a gripping portion (45).

10. The medical targeting device (27) of claim 1, further including an inwardly extending depression (49) formed at said distal end (31), wherein said depression (49) at least partially surrounds said bore (35, 35").

11. The medical target device (27) of claim 1, wherein said contrast agent is a radioactive isotope.

12. The medical targeting device (27) of claim 11, wherein said contrast agent is fluro-deoxygluscose.

13. The medical targeting device (27) of claim 11, wherein the contrast agent is technetium 99.

14. The medical targeting device (27) of claim 3, wherein said intersecting bores include a generally laterally disposed bore and a generally axial disposed bore.

15. The medical targeting device (27) of claim 14, wherein said generally laterally disposed bore has at least one closed end.

16. The medical targeting device (27) of claim 14, wherein said generally axial disposed bore extends substantially the length of said body.

17. The medical targeting device (27) of claim 16, wherein said generally laterally disposed bore is positioned adjacent said proximal end.

18. A method of preparing a medical targeting device (27) for use in a target confirmation procedure, the medical targeting device (27) comprising a localizing obturator that has at least one bore (35, 35', 35") formed in a distal end thereof; the method being **characterised by** introducing a contrast agent into said bore (35, 35', 35") such that a portion of said contrast agent is retained within a reservoir formed by said bore (35, 35', 35"); wherein said introducing step is accomplished either by dipping the distal end of said localizing obturator into said contrast agent such that contrast agent is wicked into said bore (35, 35', 35"), or by injecting contrast agent into said bore.

## Patentansprüche

1. Medizinische Zielvorrichtung (27) für ein Zielbestätigungsverfahren, umfassend:
ein längliches Körperelement (29), das durch ein distales Ende (31) und ein proximales Ende (33) definiert wird;
wobei das distale Ende (31) mindestens eine darin verlaufende Bohrung (35, 35', 35") enthält;
**dadurch gekennzeichnet, dass** die Bohrung ein flüssiges Kontrastmittel enthält, das in die Bohrung (35, 35', 35") aufgrund von Dochtwirkung oder Injizieren aufgenommen worden ist.

2. Medizinische Zielvorrichtung (27) nach Anspruch 1, wobei das distale Ende (31) zwei darin hergestellte Bohrungen (35', 35") enthält.

3. Medizinische Zielvorrichtung (27) nach Anspruch 2, wobei sich die Bohrungen (35', 35") schneiden.

4. Medizinische Zielvorrichtung (27) nach Anspruch 3, wobei sich die Bohrungen (35', 35") in einem Winkel von etwa 90° schneiden.

5. Medizinische Zielvorrichtung (27) nach Anspruch 4, wobei die Bohrungen (35', 35") einen kreuzförmigen Durchgang bilden.

6. Medizinische Zielvorrichtung (27) nach Anspruch 4, wobei die Bohrungen (35', 35") einen T-förmigen Durchgang bilden.

7. Medizinische Zielvorrichtung (27) nach Anspruch 1, wobei eine Bohrung (35") im Wesentlichen über die Länge des Körpers (29) verläuft.

8. Medizinische Zielvorrichtung (27) nach Anspruch 7, die weiterhin einen Stopfen umfasst, mit dem die Bohrung (35, 35', 35") selektiv verschlossen werden kann, der geschlossen wird, so dass das Kontrastmittel in der medizinischen Zielvorrichtung eingeschlossen ist.

9. Medizinische Zielvorrichtung (27) nach Anspruch 1, wobei das proximale Ende (33) einen Greifabschnitt (45) umfasst.

10. Medizinische Zielvorrichtung (27) nach Anspruch 1, die zudem eine an dem distalen Ende (31) hergestellte, einwärts verlaufende Einsenkung (49) umfasst, die die Bohrung (35, 35") zumindest zum Teil umgibt.

11. Medizinische Zielvorrichtung (27) nach Anspruch 1, wobei das Kontrastmittel ein radioaktives Isotop ist.

12. Medizinische Zielvorrichtung (27) nach Anspruch 11, wobei das Kontrastmittel Fluordesoxyglucose ist.

13. Medizinische Zielvorrichtung (27) nach Anspruch 11, wobei das Kontrastmittel Technetium 99 ist.

14. Medizinische Zielvorrichtung (27) nach Anspruch 3, wobei die sich schneidenden Bohrungen eine im Großen und Ganzen lateral angeordnete Bohrung und eine im Großen und Ganzen axial angeordnete Bohrung umfassen.

15. Medizinische Zielvorrichtung (27) nach Anspruch 14, wobei die im Großen und Ganzen lateral angeordnete Bohrung mindestens ein geschlossenes Ende hat.

16. Medizinische Zielvorrichtung (27) nach Anspruch 14, wobei die im Großen und Ganzen axial angeordnete Bohrung sich im Wesentlichen über die Länge des Körpers erstreckt.

17. Medizinische Zielvorrichtung (27) nach Anspruch 16, wobei die im Großen und Ganzen lateral angeordnete Bohrung in der Nähe des proximalen Endes ist.

18. Verfahren zur Herstellung einer medizinischen Zielvorrichtung (27) für die Verwendung bei einem Zielbestätigungsverfahren, wobei die medizinische Zielvorrichtung (27) einen Lokalisierungsobturator umfasst, der mindestens eine Bohrung (35, 35', 35") in einem distalen Ende besitzt, wobei das Verfahren **dadurch gekennzeichnet ist, dass** man ein Kontrastmittel in die Bohrung (35, 35', 35") so einbringt, dass ein Teil des Kotrastmittels in einem durch die Bohrung (35, 35', 35") gebildeten Reservoir zurückgehalten wird, wobei der Einbringschritt durchgeführt wird, indem man entweder das distale Ende des Lokalisierungsobturators in das Kontrastmittel so eintaucht, dass das Kontrastmittel durch Dochtwirkung in die Bohrung (35, 35', 35") gelangt, oder indem man Kontrastmittel in die Bohrung injiziert.

## Revendications

1. Dispositif (27) médical de ciblage pour une procédure de confirmation de cible, comprenant :
un élément (29) oblong, formant corps, défini par une extrémité (31) distale et par une extrémité (33) proximale ;
dans lequel l'extrémité (31) distale comprend au moins un alésage (35, 35', 35 ") s'y étendant ;
**caractérisé en ce que** l'alésage contient un agent de contraste fluide qui est reçu dans l'alésage (35, 35', 35 ") par l'un d'un effet de mèche ou d'injection.

2. Dispositif (27) médical de ciblage suivant la revendication 1, dans lequel l'extrémité (31) distale comprend deux alésages (35', 35") qui y sont formés.

3. Dispositif (27) médical de ciblage suivant la revendication 2, dans lequel les alésages (35', 35") sont en intersection l'un avec l'autre.

4. Dispositif (27) médical de ciblage suivant la revendication 3, dans lequel les alésages (35', 35") sont en intersection l'un avec l'autre suivant un angle d'environ 90°.

5. Dispositif (27) médical de ciblage suivant la revendication 4, dans lequel les alésages (35', 35") forment un passage en forme de croix.

6. Dispositif (27) médical de ciblage suivant la revendication 4, dans lequel les alésages (35', 35") forment un passage en forme de t.

7. Dispositif (27) médical de ciblage suivant la revendication 1, dans lequel un alésage (35") s'étend sensiblement sur la longueur du corps (29).

8. Dispositif (27) médical de ciblage suivant la revendication 7, comprenant en outre un tampon pour sceller sélectivement l'alésage (35, 35', 35") en le fermant de manière à sceller l'agent de contraste dans le dispositif médical de ciblage.

9. Dispositif (27) médical de ciblage suivant la revendication 1, dans lequel l'extrémité (33) proximale comprend une partie (45) de préhension.

10. Dispositif (27) médical de ciblage suivant la revendication 1, comprenant en outre une dépression (49) s'étendant vers l'intérieur et forgée à l'extrémité (31) distale, la dépression (49) entourant au moins en partie l'alésage (35', 35").

11. Dispositif (27) médical de ciblage suivant la revendication 1, dans lequel l'agent de contraste est un isotope radioactif.

12. Dispositif (27) médical de ciblage suivant la revendication 11, dans lequel l'agent de contraste est le fluoro-désoxyglucose.

13. Dispositif (27) médical de ciblage suivant la revendication 11, dans lequel l'agent de contraste est le technétium 99.

14. Dispositif (27) médical de ciblage suivant la revendication 3, dans lequel les alésages qui sont en intersection comprennent un alésage disposé d'une manière générale latéralement et un alésage disposé d'une manière générale axialement.

15. Dispositif (27) médical de ciblage suivant la revendication 14, dans lequel l'alésage disposé d'une manière générale latéralement a au moins une extrémité fermée.

16. Dispositif (27) médical de ciblage suivant la revendication 14, dans lequel l'alésage disposé d'une manière générale axialement s'étend sensiblement sur la longueur du corps.

17. Dispositif (27) médical de ciblage suivant la revendication 16, dans lequel l'alésage disposé d'une manière générale latéralement est placée à proximité de l'extrémité proximale.

18. Procédé de production d'un dispositif (27) médical de ciblage à utiliser dans un processus de confirmation de cible, le dispositif (27) médical de ciblage comprenant un obturateur de localisation, qui a au moins un alésage (35, 35', 35") formé dans son extrémité distale, le procédé étant **caractérisé en ce qu'**on introduit un agent de contraste dans l'alésage (35, 35', 35") de manière à ce qu'une partie de l'agent de contraste soit retenue dans un réservoir formé par l'alésage (35, 35', 35") , le stade d'introduction étant accompli soit en trempant l'extrémité distale de l'obturateur de localisation dans l'agent de contraste de manière à ce que l'agent de contraste soit pris par effet de mèche dans l'alésage (35, 35', 35"), soit en injectant de l'agent de contraste dans l'alésage.
